# EUROPEAN PATENT SPECIFICATION

(11) **EP 3 932 536 B1**
(45) Date of publication and mention of the grant of the patent: **05.03.2025**
(21) Application number: 20183796.0
(22) Date of filing: 02.07.2020
(51) Int. Cl.: B01J 13/06, B01J 13/22, A01N 25/28, A61K 8/11, C09B 67/02, C11D 3/50, F28D 20/02

(54) **IMPROVED MICROCAPSULES AND METHOD FOR THE PRODUCTION THEREOF**
VERBESSERTE MIKROKAPSELN UND VERFAHREN ZUR HERSTELLUNG DAVON
MICROCAPSULES AMÉLIORÉES ET LEUR PROCÉDÉ DE PRODUCTION

(43) Date of publication of application: 05.01.2022
(73) Proprietor: Follmann GmbH & Co. KG, 32423 Minden (DE)
(72) Inventor: Heemeier, Tanja, 32427 Minden (DE); Mues, Daniel, 31633 Leese (DE)
(74) Representative: König Szynka Tilmann von Renesse Patentanwälte Partnerschaft mbB Düsseldorf

(56) References cited:
- WO-A1-2009/120526
- WO-A1-2018/115330
- WO-A1-2019/243426
- WO-A1-2019/243427
- FR-A1- 2 855 074
- US-A1- 2020 114 328
- DATABASE WPI Week 201995, Derwent World Patents Index; AN 2019-96927U, XP002801207

## Description

The present invention pertains to a method for producing microcapsules, in particular biodegradable core-shell microcapsules comprising core material and shell material, microcapsules produced therewith as well as compositions comprising them.

Their uses are also disclosed.

Microcapsules allow the manufacture of products and applications with added value in all parts of industries including healthcare and personal care products, agriculture, material science and construction. In homecare products, for example laundry products, microcapsules are frequently used for achieving long lasting freshness, extending the scent of perfume oil or controlled release of the encapsulated substances (core material). Various mechanisms for a controlled release of the core material exist, for example mechanical stress, change of temperature or chemical impacts (pH shift, UV etc.). Many core-shell microcapsules, in particular, microcapsules with high stability have shells containing synthetic resins, such as melamine-formaldehyde resins, urea-formaldehyde resins or phenol-formaldehyde resins.

WO 2009/120526 A1 discloses a method for producing calcium carbonate microcapsules comprising a perfume comprising the steps of providing an emulsion comprising calcium carbonate and a perfume and adding a calcium chloride and a sodium carbonate solution.

Microcapsules based on synthetic resins contribute to the environmental burden of microplastic. Solid phase materials are considered as microplastic if the particles are not soluble in water and consist of plastics with a particulate size of 5 millimetres or smaller. Microplastic particles endanger the ecosystem on a long-term basis, including impacts on human health.

It is thus an objective of the present invention to provide a process for the manufacture of environmentally friendly microcapsules. In particular such microcapsules should have a high quality in particular in terms of stability and impermeability in order to fulfill the needs of a long lasting and controlled release of the core material comprised therein.

### SUMMARY OF THE INVENTION

This problem is solved by a method according to claim 1. Particularly advantageous embodiments of the invention are subject matter of depended or further independent claims.

The method according to the invention is generally based on the concept of microencapsulation of an emulsion that is stabilized by solid particles which adsorb onto the interface between two phases known as "Pickering emulsion" of an inorganic material, for example calcium carbonate (CaCO₃).

However, the known methods of producing microcapsules are either not suitable for industrial production and/or do not lead to a satisfactory quality of the resulting microcapsules. Wang et al. reported about preparing core-shell capsules based on CaCO₃ by encapsulating sunflower oil via Pickering emulsion templates prepared by a two-step process by inserting CO₂ gas into a reaction vessel under pressure ("Preparation of core-shell CaCO3 capsules via Pickering Emulsion templates", Journal of Colloid and Interface Science, 372, (2012) 24-31).

The inventors have found that starting the process of encapsulation with an inorganic material such as CaCO₃ in presence of a polymer which is soluble in the emulsion or suspension, leads to microcapsules of high quality. The invention enables an effective industrial production of microcapsules, which are satisfactory in particular in terms of stability and impermeability. These capsules do not require the use of synthetic resins, and therewith fulfill the need for more environmentally friendly microcapsules for industrial needs. Environment-friendly (eco-friendly) microcapsules are in particularly those which are biodegradable and/or comprising biopolymers and/or contain a low amount or even no toxic or hazardous substances. Preferred microcapsules of the invention do not contribute substantially to the microplastic contamination.

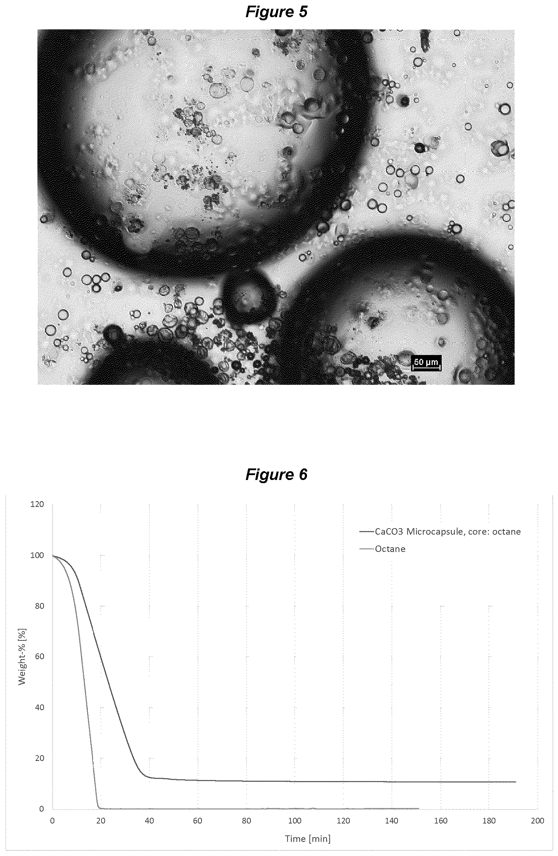

Microcapsules obtained/obtainable by the method of the invention exhibit good resistance against evaporation of the core material when the capsules are in the dry state as well as good resistance against destabilization of the capsules even if stored in air, e.g. (see leakage test, Fig. 6). Moreover the release profiles can be adjusted by the used shell material(s) - their compositions and thickness.

It was found that small microcapsules with a size of d(90%) < 50 µm in water could be prepared by the method according to the invention. They are obtainable by adjusting the mixing intensity of the emulsion or suspension, especially by stirring. Furthermore small multilayer microcapsules could be prepared by using small template microcapsules.

The microcapsules according to the invention comprise a core material. The chemical nature and structure thereof are not specifically limited. It can be a single substance or a mixture of substances. A hydrophobic substance or a mixture of substances comprising at least one hydrophobic substance is preferred. The core material can also comprise a microcapsule (hereinafter also called "template microcapsule"). The use of template microcapsules results in multilayer microcapsules.

Advantageously the template microcapsule is also a core-shell microcapsule. In one preferred embodiment the shell material of the template microcapsule (also called hereinafter the "inner shell material" of the multilayer microcapsule) comprises a synthetic resin. Various methods and resin forming polymers and prepolymers for the production of such microcapsules are known to the skilled person. The impermeability of the multilayer microcapsules can be increased by using an inner shell based on a synthetic resin. Hence, such multilayer microcapsules are particularly advantageous in terms of high impermeability (conferred by the inner shell) and high mechanical stability (conferred by the outer shell). In view of the mechanical stability conferred by the outer shell, the thickness of the inner shell can be reduced. Preferably the amount of used monomers for producing the inner shell is reduced, more preferred is reduced by at least 70 %. Therewith the overall content of the synthetic resin of such multilayer microcapsule is limited, which substantially reduces their environmental impact.

Microcapsules obtained by the method of the invention exhibit excellent resistance against evaporation of the core materials ingredient when the capsules are in the dry state as well as excellent resistance against destabilization of the capsules in air, e.g. due to storage.

The inventors have found that the method according to the invention results to stable but at the same time environment-friendly microcapsules.

Moreover, the method according to the invention allows the production of a (dry-)stable inorganic microcapsule without the use of a mandatory template.

Furthermore, the method according to the invention allows a fast and reliable production of microcapsules via a two-phase system, preferably via the Pickering emulsion - even in industrial dimensions. For example, the processes described in the state of the art are often related to issues due to high efforts in producing (dry-)stable CaCO₃ containing shells for microcapsules which are stable at the same time. In the production process of these microcapsules, the mandatory step of adding CO₂ gas to the dispersion of calcium salts and the core material (and working under pressure) is now obsolete.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only.

### BRIEF DESCRIPTION OF THE FIGURES

**Fig. 1** is a microscopic image of CaCO₃ microcapsules obtained by drying slurry on a carrier plate - the shell structure is shown by the fragments after targeted mechanical destruction of some microcapsules. (by confocal LASER-scan microscopy).
**Fig. 2** is a microscopic image of dry-stable CaCO₃ microcapsules produced by a method according to the invention; shown here are microcapsules dried onto filter paper.
**Fig. 3** is a microscopic image dried capsules on a microscope slide. The surface of the capsules shows a clear structure.
**Fig. 4** is a microscopic image of microcapsules 20 seconds after addition of 1 % acetic acid. CO₂ gas bubbles (large bubbles in foreground) are formed by the reaction of calcium carbonate with acetic acid. The structuring of the capsules surface is significantly reduced.
**Fig. 5** is a microscopic image of a microcapsule 180 seconds after addition of 1 % acetic acid. CO₂ development has continued to increase (increasing bubble sizes). The capsule shells have been completely dissolved. Only free oil droplets are still present here; collapsed cascades are visible.
**Fig. 6** is a graphic showing the results of a thermal analysis of microcapsules encapsulating octane showing core material loss as a function of temperature.

### DETAILED DESCRIPTION OF THE INVENTION

The present invention provides an improved method for producing environmentally friendly microcapsules. Moreover, the present invention provides microcapsules obtainable by the method according to the invention, in particular multilayer microcapsules which are suitable for all kinds of industrial application, in particular in personal care and home care products. The microcapsules preferably comprise at least one hydrophobic compound (e.g. oil) or at least one template microcapsule as core material.

In one embodiment of the invention a method is disclosed for producing microcapsules with core material and shell material comprising following steps,
a) providing an aqueous two-phase system comprising at least one salt, which is at least partially not dissolved in this two-phase system and a core material, which is one phase of the two-phase system,
b) adding two solutions i) and ii) in the presence of at least one polymer, which is at least partially solved in solution i) and/or ii), wherein solution i) contains at least one salt of a cation of the salt from step a) and solution ii) contains at least one salt of an anion of the salt from step a), characterized in that the polymer in step b) is a celluloseether, preferably carboxymethylcellulose or a salt thereof, more preferably sodium carboxymethylcellulose.

The two-phase system in step a) of the present invention is known as Pickering emulsion/suspension. A "two-phase system" is according to the invention a liquid-liquid system, wherein the two liquids are immiscible or at least partially immiscible and therefore separating from each other by differences in density and/or chemical nature (e.g. hydrophobic or hydrophilic character), or a liquid-solid system wherein the two phases are immiscible due to their differing aggregate states.

A "Pickering emulsion" is an emulsion that is stabilized by solid particles (e.g. colloidal silica or calcium carbonate) which adsorb onto the liquid-liquid interface between two phases. If one phase is a solid, e.g. microparticles/microcapsules which are suspended in a liquid phase, the solid stabilizing particles (e.g. colloidal silica or calcium carbonate) adsorb onto the liquid-solid interface; it can be named as a "Pickering suspension".

Step b) represents the step of growth/thickening of the shell material (by a crystallization process of the salt).

The term "phase" describes a spatial region in which material properties, such as density, refractive index, the aggregate state or chemical composition, are homogeneous. A phase is any homogeneous part of a system.

In an embodiment of the invention the method is characterised in that the two solutions i) and ii) in step b) are added in parallel.

The shell material of suitable microcapsules according to the invention can be selected from natural, semi-synthetic or synthetic materials or a mixture thereof.

Natural shell materials are inorganic materials e.g. salts like carbonates, especially calcium carbonate or other natural shell materials like gum arabic, agar-agar, agarose, maltodextrins, alginic acid or its salts, e.g. sodium or calcium alginate, fats and fatty acids, cetyl alcohol, collagen, chitosan, lecithin, gelatin, albumin, shellac, polysaccharides such as starch or dextran, polypeptides, protein hydrolysates, sucrose and waxes.

Semi-synthetic shell materials are chemically modified celluloses, especially cellulose esters and ethers of cellulose, e.g. cellulose acetate, ethyl cellulose, hydroxypropyl cellulose, hydroxypropyl methyl cellulose and carboxymethyl cellulose, as well as starch derivatives, especially starch ethers and esters.

Synthetic shell materials are (co)polymers such as polyacrylates, polymethacrylates, polyamides, polyvinyl alcohol or polyvinyl pyrrolidone.

In an embodiment, the salt in step a) is at least 50 wt% not dissolved in the two-phase system at room temperature, preferably at least 75 wt%, more preferred at least 90 wt%, most preferred at least 95 wt%, based on the used amount of inorganic material in step a).

"Room temperature" means by the disclosure of the present invention a temperature of 20 °C (293.15 Kelvin).

In a further embodiment, the salt in step a) has solubility in water of less than (<) 100 mg/L at room temperature, preferred less then (<) 50 mg/L.

In a preferred embodiment the salt of step a) is selected from the group consisting of calcium carbonate, calcium phosphate, calcium sulfate, calcium oxalate, magnesium carbonate, strontium carbonate, strontium sulphate, strontium oxalate, barium carbonate, barium sulfate, calcium fluoride, (modified) silica or mixtures thereof, preferably calcium carbonate, magnesium carbonate, barium carbonate or mixtures thereof, particularly preferably calcium carbonate.

In a more preferred embodiment the salt in step a) is calcium carbonate.

Most preferred is a mixture of hydrophilic and hydrophobic calcium carbonate.

"Hydrophilic" surfaces (e.g. of calcium carbonate particles) have a strong affinity to water and spreading of water on such surfaces are preferred. The degree of hydrophilicity of the substance can be measured by measuring the contact angle between the liquid and solid phases - for example the average water contact angle (WCA). Hydrophobic materials are known as non-polar materials with a low affinity to water, which makes them water repelling. A contact angle of less than 90° indicates hydrophilic interaction where as an angle greater than 90° indicates a hydrophobic interaction.

The water contact angle (WCA) can be measured with a Kruess DSA-100 contact angle analyzer. The measurements can be performed on the prepared powders compressed into discs using 5 µL water droplet volumes and the contacted angle can be determined from the profile of the droplets that are fully separated from the pump syringe needle tip.

In a preferred embodiment the particle size of the calcium carbonate is between 0,001 µm and 1 µm, more preferred between 0,01 µm and 0,5 µm, most preferred between 0,06 and 0,2 µm.

In another embodiment the salt in solution i) or in solution ii) of step b) is at least 40 wt% soluble at room temperature, preferred at least 65 wt%, more preferred at least 80 wt%, most preferred at least 95 wt%, based on the used amount of salt in solution i).

In a preferred embodiment, the salt in solution i) or in solution ii) of step b) has a solubility in water of more than (>) 1 g/L at room temperature, preferred more then (>) 5 g/L, more preferred more then (>) 10 g/L.

According to a more preferred embodiment of the invention the salt in solution i) is selected from the group consisting of lithium carbonate, sodium carbonate, sodium oxalate, potassium carbonate, potassium oxalate, rubidium carbonate, cesium carbonate, sodium sulfate, potassium sulfate, magnesium sulfate, trisodium phosphate, tripotassium phosphate, amine fluoride, potassium fluoride or mixtures thereof, preferably lithium carbonate, sodium carbonate, potassium carbonate or mixtures thereof, particularly preferably potassium carbonate.

In a more preferred embodiment the salt in solution ii) is selected from the group consisting of calcium formate, barium formate, magnesium formate, calcium acetate, barium acetate, magnesium acetate, calcium propanoate, barium propanoate, magnesium propanoate, calcium chloride, strontium chloride, strontium sulfate, strontium phosphate or mixtures thereof, preferably calcium formate, barium formate, calcium acetate, barium acetate or mixtures thereof, particularly preferably calcium formate and/or calcium acetate.

Suitable polymers of step b) are selected from the group consisting of celluloseether like carboxymethyl cellulose or salts thereof, preferably sodium carboxymethyl cellulose.

Suitable sodium carboxymethyl cellulose according to the invention has an average molecular weight between 10.000 g/mol and 500.000 g/mol, preferably between 75.000 g/mol and 300.000 g/mol.

Preferred sodium carboxymethyl cellulose has a viscosity at room temperature below 100 cP in a 2% aqueous solution.

More preferred the sodium carboxymethyl cellulose has a substitution degree of 0.82 to 0.95, most preferred of 0.75 to 0.90.

Steps a) and b) of the method according to the invention are preferably performed under stirring. In a preferred embodiment the stirring speed in step b) is adjusted between 600 and 3000 rpm, preferably between 1000 and 2000 rpm and is reduced to 300 to 500 rpm before step b) occurs. For step b) it is preferred to have a stirring speed between 300 to 500 rpm.

The indicated stirring speeds herein refer to a 1 liter batch e.g. in a common laboratory dissolver. The person skilled in the art knows how to adjust the stirring speed according to the reaction vessel used (e.g. larger reaction vessel requires higher stirring speeds).

In a preferred embodiment of the invention, the core material is at least one hydrophobic ingredient or at least one template microcapsule.

In a preferred embodiment of the present invention the core material comprises at least one template microcapsule. The template microcapsule is preferably a core-shell microcapsule, comprising e.g. at least one hydrophobic ingredient. The use of template microcapsules as core material according to the invention enables the production of multilayer microcapsules. The shell material of the template microcapsules/s preferably contains a resin, a natural polymer or a mixture thereof. The shell of the template microcapsule forms the inner shell of the microcapsule according to the invention.

The hydrophobic substance is preferably selected from the group comprising alcohols, perfume oils, care substances, natural and/or synthetic oils, silicone oils, pesticides, biocides, pigments, phase change materials (PCM), fertilizer, adhesives, insecticides, solvents, lubricants, dyes and cooling substances or mixtures thereof.

"Hydrophobic ingredient" means according to the invention any ingredient or material or substance or mixtures thereof which is/are mostly of hydrophobic nature. Hydrophobes tend to be nonpolar and, thus, prefer other neutral molecules and nonpolar solvents. Because water molecules are polar, hydrophobes do not dissolve well or not at all among them.

The template microcapsule comprises core material, which denotes the inner core material of the microcapsules according to the invention and shell material, which denotes the inner shell material of the microcapsules according to the invention, wherein the shell material preferably comprises a natural polymer, a resin or a mixture thereof.

In a preferred embodiment the inner core material can be a hydrophobic ingredient.

In a further preferred embodiment the natural polymer of the inner shell material is selected from the group consisting of gum arabic, chitosan, gelantine, pectine, chitin, glycogen, cellulose, xanthan gum or mixtures thereof.

In a more preferred embodiment the inner shell material is a resin. The resin preferably can be produced (version A) by a condensation of thermosetting resins and a subsequent formation of microcapsules using aldehydes, preferably formaldehyde. A process for the preparation of such microcapsules is disclosed e.g. in WO 2009/015872 A1.

It is also preferred to use a resin which can be produced
(version B) by reacting
a) at least one aromatic alcohol or ethers or esters thereof and
b) at least one aldehyde component that comprises at least two carbon atoms per molecule, and
c) in the presence of at least one homopolymer or copolymer of 2-acrylamido-2-methyl-propane sulfonic acid or salts thereof.

Suitable (in version B) as an aromatic alcohol a) are compounds selected from phenol, cresol (o-, m- and p-cresol), naphthol (α- and β-naphthol), thymol, pyrocatechol, resorcinol, hydroquinone and 1,4-naphthohydroquinone, phloroglucinol, pyrogallol, or hydroxyhydroquinone.

Suitable (in version B) as an aldehydic component b) are compounds selected from valeraldehyde, capronaldehyde, caprylaldehyde, decanal, succindialdehyde, cyclohexanecarboxaldehyde, cyclopentanecarboxaldehyde, 2-methyl-1-propanal, 2-methylpropionaldehyde, acetaldehyde, acrolein, aldosterone, antimycin A, 8'-apo-β-carotene-8'-al, benzaldehyde, butanal, chloral, citral, citronellal, crotonaldehyde, dimethylaminobenzaldehyde, folinic acid, fosmidomycin, furfural, glutaraldehyde, glyceraldehyde, glycolaldehyde, glyoxal, glyoxylic acid, heptanal, 2-hydroxybenzaldehyde, 3-hydroxybutanal, hydroxymethylfurfural, 4-hydroxynonenal, isobutanal, isobutyraldehyde, methacrolein, 2-methylundecanal, mucochloric acid, N-methylformamide, 2-nitrobenzaldehyde, nonanal, octanal, oleocanthal, orlistat, pentanal, phenylethanal, phycocyanin, piperonal, propanal, propenal, protocatechuic aldehyde, retinal, salicylaldehyde, secologanin, streptomycin, strophanthidin, tylosin, vanillin, or cinnamaldehyde.

In a preferred embodiment (in version B) the molar ratio of the aromatic alcohol a) to the aldehydic component b), which comprises at least two carbon atoms per molecule, is between 1:2 and 1:3.5, preferably between 1:2.4 and 1:2.8, and is particularly preferably between 1:2.6.

In a further embodiment of the method according to the invention at least one surfactant may be used during the production of the template microcapsules. Preferably, the surfactant is selected from the group consisting of sulfonated lignin, natural dispersants like quillaja saponin, lecithins, gum arabic, pectin, carrageenan, chitosan, chondroitin sulfate, cellulose gum, physically or chemically modified starch, whey protein, pea protein, egg white protein, silk protein, gelatin of fish, proteins of porcine or bovine origin, ester gum, fatty acids or mixtures thereof, sulfonated lignin is preferred.

This process according to version B is described in detail in WO 2011/110368 A1.

In a further embodiment of the method according to the invention at least one surfactant may be used during the production of the template microcapsules (version A or B). Preferably, the surfactant is selected from the group consisting of sulfonated lignin, natural dispersants like quillaja saponin, lecithins, gum arabic, pectin, carrageenan, chitosan, chondroitin sulfate, cellulose gum, physically or chemically modified starch, whey protein, pea protein, egg white protein, silk protein, gelatin of fish, proteins of porcine or bovine origin, ester gum, fatty acids or mixtures thereof, preferably is sulfonated lignin.

Suitable as protective colloids in the production of microcapsules are AMPS-copolymers described in detail in WO 2010/102830 A1 Examples of AMPS-copolymers are AMPS/hydroxyethylmethacrylate-copolymer, AMPS/hydroxyethylacrylate-copolymer, AMPS/hydroxypropylmethacrylate-copolymer, AMPS/hydroxypropylacrylate-copolymer, AMPS/hydroxybuylmethacrylate-copolymer, AMPS/hydroxybutylacrylate-copolymer, AMPS/polyethylene glycol monomethacrylate-copolymer, AMPS/polypropylene glycol monomethacrylate-copolymer, AMPS/polypropylene glycol monoacrylate-copolymer, AMPS/methoxy-polyethylene glycol monomethacrylate-copolymer, AMPS/methoxy-polyethylene glycol monoacrylate-copolymer, AMPS/hydroxyethylmethacrylate-copolymer, AMPS/hydroxyethylacrylate-copolymer, AMPS/hydroxypropylmethacrylate-copolymer, AMPS/hydroxypropylacrylate-copolymer, AMPS/hydroxybutylmethacrylate-copolymer, AMPS/hydroxybutylacrylate-copolymer, AMPS/polyethylene glycol monomethacrylate-copolymer, AMPS/polypropylene glycol monomethacrylate-copolymer, AMPS/polypropylene glycol monoacrylate-copolymer, AMPS/methoxy-polyethylene glycol monomethacrylate-copolymer, AMPS/methoxy-polyethylene glycol monoacrylate-copolymer.

Particularly a microcapsule according to the invention is most preferred, wherein the inner shell material (the shell material of the template microcapsule) comprises a synthetic polymer, especially preferred an aminoplastic and/or phenoplastic resin and the shell material (the outer shell material of the microcapsules) comprises at least one biopolymer, especially preferred comprises gum arabic and chitosan.

In a preferred embodiment, microcapsules produced by a method according to the invention have a low content of synthetic polymer(s).

The "content of synthetic polymer(s)" (abbreviated to: CSP) is the ratio of synthetic polymer(s) to encapsulated core material. It describes the amount of synthetic polymer (e.g. in gram, g) used/needed to encapsulate a certain amount of core material (e.g. in gram, g) - the quotient of synthetic polymer (abbreviated to: SP) to core material (abbreviated to: CM).

As an example, commercially available phenolplastic microcapsules for laundry need at least 30 g synthetic polymer(s) (SP) to encapsulate 100 g of core material (CM) (to deliver stable microcapsules, which are stable even in harsh conditions like softener or detergents); CSP = 0.3. In one method according to the invention the produced multilayer microcapsules need less than 15 g synthetic polymer(s) (SP) per 100 g core material (CM), preferably less than 10 g synthetic polymer(s) per 100 g core material; CSP = 0.1.

As a consequence, producing microcapsules by a method according to the invention reduces the content of synthetic polymer(s) by 30 %, preferred by 60 %, more preferred by 75 %, most preferred by 80 %.

Microcapsules according to one preferred embodiment have a content of synthetic polymer(s) (CSP) less than or equal to (≤) 0.15, wherein the CSP is the quotient of the amount of synthetic polymer(s) encapsulating an amount of core material.

In a more preferred embodiment the multilayer microcapsules have a CSP of less than or equal to (≤) 0.15, preferred less than (<) 0.1, more preferred less than (<) 0.08.

The value for CSP can be determined by any method known by a skilled person in the art.

Preferably the value for CSP can be determined in a first step, wherein the amount of synthetic polymer (SP) is determined by subtracting the amount of not reacted / unreacted monomers or other educts that were used to create (inner) shell material comprising synthetic polymer(s) during the production process of the microcapsules from the amount of monomers or other educts that were originally used to create (inner) shell material comprising synthetic polymer(s) during the production process of the microcapsules. In a second step, the amount of core material (CM) is determined by subtracting the amount of free / not encapsulated core material (core material which is not encapsulated during the production process of the microcapsules) from the amount of core material originally used to be encapsulated as core material during the production process. The quotient of SP and CM delivers the value for CSP.

In an embodiment the method according to the invention may comprise a further step c) wherein the slurry obtained after step b) is dried to provide dried microcapsules.

The drying process may be any technology which is state of the art, preferably spray drying, freeze drying, drum drying and/or pulse combustion drying, more preferred spray drying.

Microcapsules obtained by a method according to the invention are also claimed.

Microcapsules according to any of the embodiments are stable in storage after drying for at least 14 days, preferably for at least 28 days, particularly preferably for at least 36 days at storage temperatures from 20 to 45 °C and at a relative air humidity of 30 to 70 %.

In a preferred embodiment of the invention the microcapsules are characterized in that the shell material comprises at least 40 wt.-% of a salt according to one of the embodiments and at least 0.1 wt.-% of a water-soluble polymer selected from the group consisting of celluloseether like carboxymethyl cellulose or salts thereof, preferably sodium carboxymethyl cellulose or polyelectrolytes like gum arabic, chitosan, xanthan gum, alginate, carrageen or pectine, more preferred the soluble polymer consist of carboxymethyl cellulose.

In a further preferred embodiment of the invention, the shell material comprises at least 40 wt.-% of a salt selected from the group consisting of calcium carbonate, magnesium carbonate, barium carbonate or mixtures thereof, particularly preferably calcium carbonate and at least 0.1 wt.-% of a soluble polymer is a cellulose, preferably is carboxymethyl cellulose, more preferably sodium carboxymethyl cellulose.

Typically, the microcapsules according to the invention have a hydrodynamic diameter d(90%) of between 1 µm and 5.000 µm, preferably between 5 µm and 1.000 µm, more preferably between 5 and 150 µm, even more preferably between 10 and 120 µm, most preferred between 10 and 100 µm, with a standard deviation of ± 30 µm (measured via DLS in deionized water at room temperature, volume-weighted evaluation).

The size of the microcapsules according to the invention could be adjusted by the mixing intensity of the emulsion or dispersion, especially by stirring (depending on the rounds per minute, rpm). Especially the size of multilayer microcapsules can be adjusted by producing and/or using correspondingly small template microcapsules.

The ratio of core material to shell material in step a) of the method according to the invention is preferably between 1:10 and 10:1, more preferred between 1:4 and 4:1, most preferred between 2:3 and 3:2.

In an embodiment according to the invention, the microcapsules open and release the core material at a pressure of at least 5 N, preferably at a pressure of at least 10 N, particularly preferably at a pressure of at least 50 N, extremely preferably at a pressure of at least 100 N. In another embodiment the microcapsules according to the invention have a pH-depending opening mechanism. The microcapsules are opened and release the core material at a pH less (<) than 7.0, preferably at a pH less (<) than 6.0.

The present disclosure also relates to the use of microcapsules obtained or obtainable by the invention. Compositions comprising them are also claimed. Such compositions or products may be either solid or liquid product. According to a particular embodiment, liquid products are preferred. Preferred products and composition are used in the "home- or personal- care" industry, e.g. laundry or cleansing compositions.

The invention will now be further described by way of examples. It will be appreciated that the invention as claimed is not intended to be limited in any way by these examples.

### EXAMPLES

### Example 1

The process of microencapsulation of at least one hydrophobic substance by a method according to the invention, based on a Pickering emulsion process, can be described as two basic steps:
**1. Creating a two-phase system:**
   1 g of precipitated calcium carbonate and 3 g fine precipitated calcium carbonate are dispersed in 80 ml of demineralized water in a 200 mL beaker (9000 rpm with the disperser/lab dissolver e.g. Ultra Turaxx^{®}). When the calcium carbonate is well dispersed, the homogenization speed/intensity is slowly decreased to 3600 rpm. Then, 18.50 g of the water-immiscible core material (e.g. any hydrophobic compounds, perfume, fragrance oil etc.) is added dropwise to the dispersion. After 30 minutes the Pickering emulsion is ready and the emulsion formation is completed.
**2. Growth/Thickening of the CaCO₃ shell (crystallization process):**
   The emulsion from the first step is provided and heated to 40 °C. The speed of the homogenizer is now at 400 rpm. Two solutions are then added to this emulsion in parallel. Solution 1 consists of 88.20 g deionized water, 9.8 g K₂CO₃ and 2 g of water-soluble carboxymethyl cellulose (CMC, with a substitution degree of more then 0.75, preferred more than 0.82). The second solution consists of 90 g deionized water and 10 g calcium chloride. Both solutions are added parallel and stepwise within the time span of 75 minutes.

Both the use of other inorganic compounds (and combinations thereof) and/or water-immiscible core material (and combinations thereof) are included in the disclosure of this invention.

### Proof of drying stability

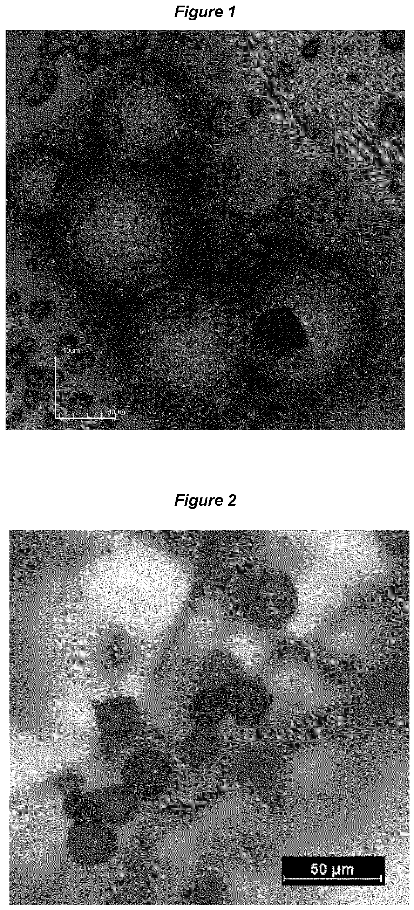

The drying stability of the microcapsules can be demonstrated by light microscopy. The following figure 1 clearly shows the dried microcapsules after spraying the slurry onto filter paper (see Fig. 1 and Fig. 2).

### Proof of pH-dependent opening

The inorganic microcapsules were brought into contact with 1% acetic acid. The behavior of the capsules in this medium was evaluated by light microscopy.

### Step 1: Fixation of the capsules on a microscope slide

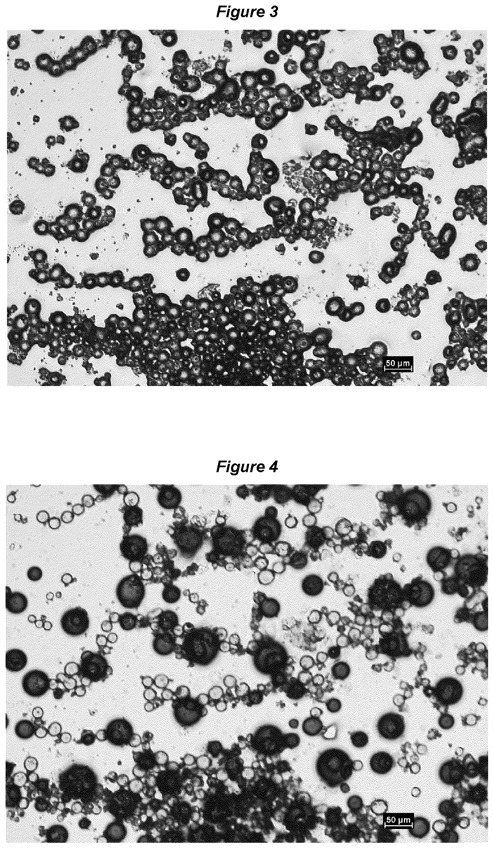

→ Microscopic image see Fig. 3
→ Observation: Dry capsules can be clearly identified in both observation ranges. The surface of the capsules shows a clear structure.

### Step 2: Addition of 1% acetic acid

### Step 2.1: Observation 20 seconds after addition of 1 % acetic acid

→ Microscopic image see Fig. 4
→ Observation: CO₂ gas bubbles are formed by the reaction of calcium carbonate with acetic acid. The structuring of the capsules surface is significantly reduced in both observation areas.

### Step 2.2: Observation 180 seconds after addition of 1 % acetic acid

→ Microscopic image see Fig. 5
→ Observation after 180 seconds: CO₂ development has continued to increase. In observation range 1 the capsule shells have been completely dissolved. Only free oil droplets are still present here. In observation area 2 collapsed cascades are visible.

### Leakage test

A thermal analysis of microcapsules (shell material: CaCO₃, core material: octane with coumarin) was performed. As comparison test a thermal analysis of pure octane without encapsulation was also performed. For the measurements a PERKINELMER TGA 8000^{™} Thermogravimetric analyzer was used. The test temperature program was for both samples the following:
1.) Holding for 1.0 min at 30 °C,
2.) Heat from 30 °C to 80 °C at 5.00 °C/min,
3.) Holding heat for 180.0 min at 80°C min

Purging gas was nitrogen with a purge gas rate of 20.0 mL/min.

Figure 6 shows the thermogramm for octane and CaCO₃ microcapsules with a core material comprising octane. In the first step, part of the water and non-encapsulated octane evaporates. The subsequent isothermal storage at 80°C corresponds to the leakage test, whereby any remaining water and non-encapsulated water will initially escape.

At the beginning of the actual leakage test, the capsule content is approx. 12.0 % from the 40th minute and decreases to 10.6 % at the end of the test. The calculated wall fraction of the microcapsules is 8.6 %, which means encapsulated octane is still present even after storage of the microcapsules under these drastic conditions.

### Example 2 - Multi-layer microcapsules

The process of microencapsulation of at least one further microcapsule (template microcapsule) via a Pickering emulsion with a salt which is at least partially not dissolved in the aqueous two-phase system, based on an emulsion/suspension process, can be described in two main steps - the formation of the further microcapsules (template microcapsules) or the simple provision of such template microcapsules followed by the encapsulation with at least one salt which is at least partially not dissolved in the aqueous two-phase system.

### a) First step - Formation of a first microcapsule (template microcapsule)

### Version A: Aminoplastic resin method

The formation of the first capsule (template microcapsule) is based on the process described in patent WO2009/015872 A1. As protective colloids, biodegradable polymers carrying sulfonic acid groups are preferred, especially the sodium, calcium and ammonium salts of lignosulfonic acid.

The microcapsule composition is obtainable by the condensation of thermosetting resins and subsequent formation of microcapsules using aldehydes, preferably formaldehyde and optionally, wherein the shell material of the capsule has been treated with an oxidizing agent (e.g. H₂O₂) prior to curing of the shell material optionally supplemented by silica or a silica derivative.

### Version B: Formaldehyde-free phenolic resin method

The formation of the first capsule (template microcapsule) is produced according to the process described in patent WO2011/110368 A2.

Those microcapsule's shells comprise a resin which is prepared by reacting a) at least one compound selected from the group consisting of a1) amines and a2) aromatic or heteroaromatic compounds which are unsubstituted or substituted by one or more substituents from the group C₁-C₂₀ alkyl, OH, OR, COOH, SH, SR, NHCOR, OCOR, halogen, aromatic, wherein R represents a C₁-C₁₀ alkyl group, with b) at least one aldehydic component containing at least two C atoms per molecule, in the presence of c) at least one copolymer containing units of 2-acrylamido-2-methyl-propanesulfonic acid or its salts (AMPS) and/or 2-acrylamido-2-methyl-propanephosphonic acid or its salts (AMPP) and units of one or more (meth)acrylates. Preferred is a1) selected from the group consisting of urea, melamine and benzoguanamine and a2) is selected from the group consisting of one or more aromatic or heteroaromatic alcohols and/or one or more aromatic or heteroaromatic carboxylic acids, more preferably selected from the group consisting of pyrocatechol, resorcinol, hydroquinone and 1 ,4-naphthohydroquinone, phloroglucinol, pyrogallol, hydroxyhydroquinone, most preferably resorcinol and phloroglucin and the b) the least one aldehydic component is selected from the group consisting of valeraldehyde, caproic aldehyde, caprylic aldehyde, decanal, succindialdehyde, cyclohexane carbaldehyde, cyclopentane carbaldehyde, 2-methyl-1-propanal, 2-methylpropionaldehyde, acetaldehyde, acrolein, aldosterone, antimycin A, 8'-Apo-ß-caroten-8'-al, benzaldehyde, butanal, chloral, citral, citronellal, crotonaldehyde, dimethylaminobenzaldehyde, folic acid, fosmidomycin, furfural, glutaraldehyde, glyceraldehyde, glycolaldehyde, glyoxal, glyoxylic acid, Heptanal, 2-hydroxybenzaldehyde, 3-hydroxybutanal, hydroxymethylfurfural, 4-hydroxynonenal, isobutanal, isobutyraldehyde, methacrolein, 2-methylundecanal, mucochloric acid, N-methylformamide, 2-nitrobenzaldehyde, nonanal, octanal, oleocanthal, orlistat, pentanal, phenylethanal, phycocyanin, piperonal, propanal, propenal, protocatechualdehyde, retinal, salicylaldehyde, secologanin, streptomycin, strophanthidin, tylosin, vanillin, cinnamic aldehyde and c) is selected from the group consisting of biopolymer or a terpolymer, which is more preferably composed of units of AMPS or AMPP, preferably AMPS, and one or more (meth)acrylates.

Preferred are template microcapsules according to one of the two mentioned methods from version A or version B. According to the invention, template microcapsules from both methods can also be used as a mixture or independently of each other for the further encapsulation according to the invention's method. In general, all capsules, particles or template microcapsules can be encapsulated by the method according to the invention.

### b) Second step - Encapsulation of the first microcapsule (template microcapsule) by a method according to the invention

The process of microencapsulation of at least one template microcapsules by a method according to the invention, based on a Pickering suspension process, can be described as two basic steps:
**1. Creating a two-phase system:**
   1 g of precipitated calcium carbonate and 3 g of fine precipitated calcium carbonate are dispersed in 80 ml of demineralized water in a 200 mL beaker (9000 rpm with the disperser e.g. Ultra Turaxx^{®}). When the calcium carbonate is well dispersed, the homogenization speed/intensity is slowly decreased to 3600 rpm. Then, the core material, here template microcapsules, are added stepwise to the dispersion. After 30 minutes the Pickering suspension is ready.
**2. Growth/Thickening of the CaCO₃ shell (crystallization process):**
   The two-phase system (suspension) from the first step is provided and heated to 40 °C. The speed of the homogenizer is now at 400 rpm. Two solutions are then added to this suspension in parallel. Solution 1 consists of 88.20 g deionized water, 9.8 g K₂CO₃ and 2 g of water-soluble carboxymethyl cellulose (CMC, with a substitution degree of more then 0.75, preferred more than 0.82). The second solution consists of 90 g deionized water and 10 g calcium formate. Both solutions are added parallel and stepwise within the time span of 15 minutes

Both the use of other inorganic compounds (and combinations thereof) and/or other core materials (and combinations thereof) are included in the disclosure of this invention.

## Claims

1. A method for producing microcapsules with core material and shell material comprising following steps,
a) providing an aqueous two-phase system comprising at least one salt, which is at least partially not dissolved in this two-phase system and a core material, which is one phase of the two-phase system,
b) adding two solutions i) and ii) in the presence of at least one polymer, which is at least partially solved in solution i) and/or ii), wherein solution i) contains at least one salt of a cation of the salt from step a) and solution ii) contains at least one salt of an anion of the salt from step a),
**characterized in that** the polymer in step b) is a celluloseether, preferably carboxymethylcellulose or a salt thereof, more preferably sodium carboxymethylcellulose.

2. The method according to claim 1, **characterized in that** the salt of step a) is selected from the group consisting of calcium carbonate, calcium phosphate, calcium sulfate, calcium oxalate, magnesium carbonate, strontium carbonate, strontium sulphate, strontium oxalate, barium carbonate, barium sulfate, calcium fluoride, modified silica or mixtures thereof, preferably calcium carbonate, magnesium carbonate, barium carbonate or mixtures thereof, particularly preferably calcium carbonate.

3. The method according to claim 1 or 2, **characterized in that** the salt in solution i) of step b) is selected from the group consisting of lithium carbonate, sodium carbonate, sodium oxalate, potassium carbonate, potassium oxalate, rubidium carbonate, cesium carbonate, sodium sulfate, potassium sulfate, magnesium sulfate, trisodium phosphate, tripotassium phosphate, amine fluoride, potassium fluoride or mixtures thereof, preferably lithium carbonate, sodium carbonate, potassium carbonate or mixtures thereof, particularly preferably potassium carbonate.

4. The method according to any of the claims 1 to 3, **characterized in that** the salt in solution ii) of step b) is selected from the group consisting of calcium formate, barium formate, magnesium formate, calcium acetate, barium acetate, magnesium acetate, calcium propanoate, barium propanoate, magnesium propanoate, calcium chloride, strontium chloride, strontium sulfate, strontium phosphate or mixtures thereof, preferably calcium formate, barium formate, calcium acetate, barium acetate or mixtures thereof, particularly preferably calcium formate and/or calcium acetate.

5. The method according to claims 1 to 4, **characterized in that** the core material is at least one hydrophobic ingredient or at least one template microcapsule.

6. The method according to claim 5, **characterized in that** the hydrophobic ingredient is selected from the group consisting of alcohols, perfume oils, care substances, natural and/or synthetic oils, silicone oils, pesticides, biocides, pigments, phase change materials (PCM), fertilizer, adhesives, insecticides, solvents, lubricants, dyes and cooling substances or mixtures thereof.

7. The method according to claim 5, **characterised in that** the template microcapsule comprises a shell material (inner shell material), which comprises a natural polymer, a resin or a mixture thereof.

8. The method according to claim 7, **characterised in that** the resin is obtained by the condensation of thermosetting resins and a subsequent formation of microcapsules using aldehydes, preferably formaldehyde and optionally, wherein the shell material of the microcapsule has been treated with an oxidizing agent preferred with H₂O₂ prior to curing of the shell material further optionally supplemented by silica or a silica derivative.

9. The method according to claim 7 or 8, **characterised in that** the resin is obtained by reacting
a) at least one aromatic alcohol or ethers or esters thereof and
b) at least one aldehyde component that comprises at least two carbon atoms per molecule, and
c) in the presence of at least one homopolymer or copolymer of 2-acrylamido-2-methyl-propane sulfonic acid or salts thereof.

10. The method according to claim 7, **characterised in that** the natural polymer is selected from the group consisting of gum arabic, chitosan, gelatine, pectine, chitin, glycogen, cellulose, xanthan gum or mixtures thereof.

11. Microcapsules obtained by a method according to any one of the claims 1 to 10, wherein the shell material comprises the polymer of step b), which is a celluloseether, preferably carboxymethylcellulose or a salt thereof, more preferably sodium carboxymethylcellulose.

12. Microcapsules according to claim 11, whereas the microcapsules have a hydrodynamic diameter d(90%) between 5 and 150 µm, preferred between 10 and 120 µm, more preferred between 10 and 100 µm, with a standard deviation of ± 30 µm

13. A composition comprising microcapsules according to any one of the claims 11 or 12.

## Patentansprüche

1. Verfahren zum Herstellen von Mikrokapseln mit Kernmaterial und Hüllmaterial, umfassend die folgenden Schritte,
a) Bereitstellen eines wässrigen Zweiphasensystems, das mindestens ein Salz, das in diesem Zweiphasensystem mindestens teilweise nicht gelöst ist, und ein Kernmaterial umfasst, das eine Phase des Zweiphasensystems ist,
b) Hinzufügen von zwei Lösungen i) und ii) in Gegenwart von mindestens einem Polymer, das mindestens teilweise in Lösung i) und/oder ii) gelöst ist, wobei Lösung i) mindestens ein Salz eines Kations des Salzes aus Schritt a) enthält und Lösung ii) mindestens ein Salz eines Anions des Salzes aus Schritt a) enthält,
**dadurch gekennzeichnet, dass** das Polymer in Schritt b) ein Celluloseether, bevorzugt Carboxymethylcellulose oder ein Salz davon, besonders bevorzugt Natriumcarboxymethylcellulose ist.

2. Verfahren nach Anspruch 1, **dadurch gekennzeichnet, dass** das Salz von Schritt a) aus der Gruppe ausgewählt ist, die aus Calciumcarbonat, Calciumphosphat, Calciumsulfat, Calciumoxalat, Magnesiumcarbonat, Strontiumcarbonat, Strontiumsulfat, Strontiumoxalat, Bariumcarbonat, Bariumsulfat, Calciumfluorid, modifiziertem Siliciumdioxid oder Mischungen davon besteht, bevorzugt Calciumcarbonat, Magnesiumcarbonat, Bariumcarbonat oder Mischungen davon, besonders bevorzugt Calciumcarbonat.

3. Verfahren nach Anspruch 1 oder 2, **dadurch gekennzeichnet, dass** das Salz in Lösung i) von Schritt b) aus der Gruppe ausgewählt ist, die aus Lithiumcarbonat, Natriumcarbonat, Natriumoxalat, Kaliumcarbonat, Kaliumoxalat, Rubidiumcarbonat, Cäsiumcarbonat, Natriumsulfat, Kaliumsulfat, Magnesiumsulfat, Trinatriumphosphat, Trikaliumphosphat, Aminfluorid, Kaliumfluorid oder Mischungen davon besteht, bevorzugt Lithiumcarbonat, Natriumcarbonat, Kaliumcarbonat oder Mischungen davon, besonders bevorzugt Kaliumcarbonat.

4. Verfahren nach einem der Ansprüche 1 bis 3, **dadurch gekennzeichnet, dass** das Salz in Lösung ii) von Schritt b) aus der Gruppe ausgewählt ist, die aus Calciumformiat, Bariumformiat, Magnesiumformiat, Calciumacetat, Bariumacetat, Magnesiumacetat, Calciumpropanoat, Bariumpropanoat, Magnesiumpropanoat, Calciumchlorid, Strontiumchlorid, Strontiumsulfat, Strontiumphosphat oder Mischungen davon besteht, bevorzugt Calciumformiat, Bariumformiat, Calciumacetat, Bariumacetat oder Mischungen davon, besonders bevorzugt Calciumformiat und/oder Calciumacetat.

5. Verfahren nach den Ansprüchen 1 bis 4, **dadurch gekennzeichnet, dass** das Kernmaterial mindestens ein hydrophober Inhaltsstoff oder mindestens eine Templat-Mikrokapsel ist.

6. Verfahren nach Anspruch 5, **dadurch gekennzeichnet, dass** der hydrophobe Inhaltsstoff aus der Gruppe ausgewählt ist, die aus Alkoholen, Parfümölen, Pflegesubstanzen, natürlichen und/oder synthetischen Ölen, Silikonölen, Pestiziden, Bioziden, Pigmenten, Phasenwechselmaterialien (PCM), Düngemitteln, Klebstoffen, Insektiziden, Lösungsmitteln, Schmierstoffen, Farbstoffen und Kühlsubstanzen oder Mischungen davon besteht.

7. Verfahren nach Anspruch 5, **dadurch gekennzeichnet, dass** die Templat-Mikrokapsel ein Hüllmaterial (inneres Hüllmaterial) umfasst, das ein natürliches Polymer, ein Harz oder eine Mischung davon umfasst.

8. Verfahren nach Anspruch 7, **dadurch gekennzeichnet, dass** das Harz durch die Kondensation von duroplastischen Harzen und einer anschließenden Bildung von Mikrokapseln unter Verwendung von Aldehyden, bevorzugt Formaldehyd, gewonnen wird und wobei das Hüllmaterial der Mikrokapsel vor dem Aushärten des Hüllmaterials mit einem Oxidationsmittel, bevorzugt H₂O₂, behandelt und ferner optional durch Siliciumdioxid oder ein Siliciumdioxidderivat supplementiert wurde.

9. Verfahren nach Anspruch 7 oder 8, **dadurch gekennzeichnet, dass** das Harz durch Reagierenlassen von Folgendem gewonnen wird:
a) mindestens einem aromatischen Alkohol oder Ethern oder Estern davon und
b) mindestens einer Aldehydkomponente, die mindestens zwei Kohlenstoffatome pro Molekül umfasst, und
c) in Gegenwart mindestens eines Homopolymers oder Copolymers von 2-Acrylamido-2-methylpropansulfonsäure oder Salzen davon.

10. Verfahren nach Anspruch 7, **dadurch gekennzeichnet, dass** das natürliche Polymer aus der Gruppe ausgewählt ist, die aus Gummi arabicum, Chitosan, Gelatine, Pektin, Chitin, Glykogen, Cellulose, Xanthangummi oder Mischungen davon besteht.

11. Mikrokapseln, die durch ein Verfahren nach einem der Ansprüche 1 bis 10 gewonnen werden, wobei das Hüllmaterial das Polymer von Schritt b) umfasst, das ein Celluloseether, bevorzugt Carboxymethylcellulose oder ein Salz davon, besonders bevorzugt Natriumcarboxymethylcellulose ist.

12. Mikrokapseln nach Anspruch 11, wobei die Mikrokapseln einen hydrodynamischen Durchmesser d(90 %) zwischen 5 und 150 µm, bevorzugt zwischen 10 und 120 µm, besonders bevorzugt zwischen 10 und 100 µm, mit einer Standardabweichung von ± 30 µm aufweisen.

13. Zusammensetzung, umfassend Mikrokapseln nach einem der Ansprüche 11 oder 12.

## Revendications

1. Procédé de production de microcapsules avec un matériau de noyau et un matériau d'enveloppe comprenant les étapes suivantes,
a) la fourniture d'un système aqueux à deux phases comprenant au moins un sel, qui est au moins partiellement non dissous dans ce système à deux phases et d'un matériau de noyau, qui est une phase du système à deux phases,
b) l'ajout de deux solutions i) et ii) en présence d'au moins un polymère, qui est au moins partiellement dissous dans la solution i) et/ou ii), dans lequel la solution i) contient au moins un sel d'un cation du sel de l'étape a) et la solution ii) contient au moins un sel d'un anion du sel de l'étape a),
**caractérisé en ce que** le polymère de l'étape b) est un éther de cellulose, de préférence la carboxyméthylcellulose ou un sel de celle-ci, idéalement la carboxyméthylcellulose de sodium.

2. Procédé selon la revendication 1, **caractérisé en ce que** le sel de l'étape a) est choisi dans le groupe constitué par le carbonate de calcium, le phosphate de calcium, le sulfate de calcium, l'oxalate de calcium, le carbonate de magnésium, le carbonate de strontium, le sulfate de strontium, l'oxalate de strontium, le carbonate de baryum, le sulfate de baryum, le fluorure de calcium, la silice modifiée ou des mélanges de ceux-ci, de préférence le carbonate de calcium, le carbonate de magnésium, le carbonate de baryum ou des mélanges de ceux-ci, de manière particulièrement préférée le carbonate de calcium.

3. Procédé selon la revendication 1 ou 2, **caractérisé en ce que** le sel dans la solution i) de l'étape b) est choisi dans le groupe constitué par le carbonate de lithium, le carbonate de sodium, l'oxalate de sodium, le carbonate de potassium, l'oxalate de potassium, le carbonate de rubidium, le carbonate de césium, le sulfate de sodium, le sulfate de potassium, le sulfate de magnésium, le phosphate trisodique, le phosphate tripotassique, le fluorure d'amine, le fluorure de potassium ou des mélanges de ceux-ci, de préférence le carbonate de lithium, le carbonate de sodium, le carbonate de potassium ou des mélanges de ceux-ci, de manière particulièrement préférée le carbonate de potassium.

4. Procédé selon l'une quelconque des revendications 1 à 3, **caractérisé en ce que** le sel dans la solution ii) de l'étape b) est choisi dans le groupe constitué par le formiate de calcium, le formiate de baryum, le formiate de magnésium, l'acétate de calcium, l'acétate de baryum, l'acétate de magnésium, le propanoate de calcium, le propanoate de baryum, le propanoate de magnésium, le chlorure de calcium, le chlorure de strontium, le sulfate de strontium, le phosphate de strontium ou des mélanges de ceux-ci, de préférence le formiate de calcium, le formiate de baryum, l'acétate de calcium, l'acétate de baryum ou des mélanges de ceux-ci, de manière particulièrement préférée le formiate de calcium et/ou l'acétate de calcium.

5. Procédé selon les revendications 1 à 4, **caractérisé en ce que** le matériau de noyau est au moins un ingrédient hydrophobe ou au moins une microcapsule modèle.

6. Procédé selon la revendication 5, **caractérisé en ce que** l'ingrédient hydrophobe est choisi dans le groupe constitué par les alcools, les huiles parfumées, les substances de soin, les huiles naturelles et/ou synthétiques, les huiles de silicone, les pesticides, les biocides, les pigments, les matériaux à changement de phase (PCM), les engrais, les adhésifs, les insecticides, les solvants, les lubrifiants, les colorants et les substances de refroidissement ou des mélanges de ceux-ci.

7. Procédé selon la revendication 5, **caractérisé en ce que** la microcapsule modèle comprend un matériau d'enveloppe (matériau d'enveloppe interne), qui comprend un polymère naturel, une résine ou un mélange de ceux-ci.

8. Procédé selon la revendication 7, **caractérisé en ce que** la résine est obtenue par condensation de résines thermodurcissables et formation ultérieure de microcapsules à l'aide d'aldéhydes, de préférence de formaldéhyde et éventuellement, dans lequel le matériau d'enveloppe de la microcapsule a été traité avec un agent oxydant préféré à H₂O₂ avant le durcissement du matériau d'enveloppe, éventuellement en outre complété par de la silice ou un dérivé de silice.

9. Procédé selon l'une des revendications 7 ou 8, **caractérisé en ce que** la résine est obtenue par réaction
a) d'au moins un alcool aromatique ou des éthers ou esters de celui-ci, et
b) d'au moins un composant aldéhyde qui comprend au moins deux atomes de carbone par molécule, et
c) en présence d'au moins un homopolymère ou copolymère d'acide 2-acrylamido-2-méthylpropane sulfonique ou de sels de celui-ci.

10. Procédé selon la revendication 7, **caractérisé en ce que** le polymère naturel est choisi dans le groupe constitué par la gomme arabique, le chitosane, la gélatine, la pectine, la chitine, le glycogène, la cellulose, la gomme xanthane ou des mélanges de ceux-ci.

11. Microcapsules obtenues par un procédé selon l'une quelconque des revendications 1 à 10, dans lesquelles le matériau d'enveloppe comprend le polymère de l'étape b), qui est un éther de cellulose, de préférence la carboxyméthylcellulose ou un sel de celle-ci, idéalement la carboxyméthylcellulose de sodium.

12. Microcapsules selon la revendication 11, dans lesquelles les microcapsules comportent un diamètre hydrodynamique d(90 %) compris entre 5 et 150 µm, de préférence entre 10 et 120 µm, de préférence encore entre 10 et 100 µm, avec un écart type de ± 30 µm.

13. Composition comprenant des microcapsules selon l'une quelconque des revendications 11 ou 12.
